# EUROPÄISCHE PATENTSCHRIFT

(11) **EP 0 324 978 B1**
(45) Veröffentlichungstag und Bekanntmachung des Hinweises auf die Patenterteilung: **24.02.1993**
(21) Anmeldenummer: 88121755.8
(22) Anmeldetag: 28.12.1988
(51) Int. Cl.: B21B 35/14

(54) **Walzwerksantrieb mit zwischen Kammwalzen und Arbeitswalzen lösbar angeordneten Gelenkspindeln**
Rolling mill drive with loosenable driving spindles arranged between gearings and work rolls
Entraînement pour laminoir à allonges d'entraînement déconnectables entre roues dentées et cylindres de travail

(30) Priorität: 22.01.1988 DE 3801749
(43) Veröffentlichungstag der Anmeldung: 26.07.1989
(73) Patentinhaber: SMS SCHLOEMANN-SIEMAG AKTIENGESELLSCHAFT, 40237 Düsseldorf (DE)
(72) Erfinder: Seidl, Karl Heinz, D-5912 Hilchenbach (DE); Pithan, Gerhard, D-5902 Netphen (DE); Stelbrink, Jürgen, D-5912 Hilchenbach (DE)
(74) Vertreter: Müller, Gerd, Dipl.-Ing.

(56) Entgegenhaltungen:
- DE-B- 1 138 286
- GB-A- 2 187 820
- US-A- 2 909 045

## Beschreibung

Die Erfindung betrifft einen Walzwerksantrieb mit zwischen Kammwalzen und Arbeitswalzen axial verschieblich angeordneten Gelenkspindeln, deren Spindelgelenk lösbar mit dem Laufzapfen wenigstens einer Walze, insbesondere der Arbeitswalze verbunden ist, wobei zumindest das mit der Arbeitswalze verbundene Spindelgelenk eine Ein- und Entkopplungsvorrichtung aufweist, die als Ring-Bajonett-Verschluß ausgebildet ist, wobei der Ring-Bajonett-Verschluß den Laufzapfen der Arbeitswalze mit der Gelenkhülse des Spindelgelenks koppelt und die Innenverzahnung der Gelenkhülse mit einer Bogenverzahnung eines auf der Spindelwelle angeordneten Gelenktreffers zusammenwirkt.

Bei herkömmlichen Walzgerüsten erfolgt der Antrieb der Arbeitswalzen, zwischen denen das Walzgut, beispielsweise ein kalt zu walzendes Metallband bearbeitet wird, in der Weise, daß mit den Laufzapfen der Arbeitswalzen Antriebsspindeln verbunden sind, die mit antreibenden Kammwalzen in Verbindung stehen, wobei die Kammwalzen über zwischengeschaltete Getriebe von geeigneten Antriebsmotoren angetrieben werden. Die Antriebsspindeln weisen an den Verbindungen zu den Arbeitswalzen bzw. zu den Kammwalzen Spindelgelenke auf, um die durch verschiedene Walzbanddicken verursachten Winkelverlagerungen auszugleichen. Da die Arbeitswalzen im gängigen Walzbetrieb hohen Anforderungen genügen müssen und wegen des auftretenden Walzenverschleißes und wegen der Anpassung an das jeweilige Walzprogramm haüfiger gewechselt werden müssen, sind besondere konstruktive Maßnahmen vorgesehen, um Arbeitswalzen und Gelenkspindeln voneinander zu trennen. Bei bekannten Trennvorrichtungen ist das Auswechseln der Walzen einschließlich der Entfernung der Zapfen, der Gleitstücke und das Vorziehen der Gelenkspindel sehr mühsam und erfordert Zeit und erheblichen Arbeitsaufwand.

Aus der US-PS 3,670,587 ist ein Vertikalwalzwerk bekannt, bei welchem das Auswechseln der Walzen in der Weise erfolgt, daß zwei Walzen jeweils voneinander fort und dann abwärts bewegt und mit ihren Laufzapfen aus den Kupplungen der Antriebsvorrichtungen herausgezogen werden. Dies erfordert jedoch aufwendige und komplizierte Vorrichtungen zur Verschiebung und axialen Bewegung der Arbeitswalzen. Wenn die Kupplungen der Antriebsvorrichtungen nicht nach dem Herausziehen des Laufzapfens in ihrer Position festgelegt werden, ist das nachfolgende Einführen der Laufzapfen der neuen Walzen in die Kupplung sehr schwierig.

Aus der DE-OS 27 33 988 ist eine Antriebsvorrichtung für vertikale Walzwerkswalzen bekannt mit einer Antriebswelle, die über ein Universalgelenk lösbar mit dem Laufzapfen einer Walze verbunden ist und bei der eine Innenwelle und eine mit dieser Innenwelle verbundene Außenwelle axial verschiebbar sind. Zum Aus- und Einkuppeln des Laufzapfens der Walzwerkswalze mit dem zugehörigen Kupplungsjoch des Universalgelenks der Antriebswalze ist eine an der Innenwelle und der Außenwelle angreifende Stellvorrichtung vorgesehen, die aus einem an der Außenwelle befestigten Druckmittelzylinder mit an der Innenwelle gehalterter Kolbenstange besteht. Durch Betätigen des Druckmittelzylinders wird die Außenwelle auf der Innenwelle verschoben und dadurch so weit verkürzt bis das Kupplungsjoch des Universalgelenks von dem Walzenzapfen abgezogen ist. Diese bekannte Abziehvorrichtung ist aufwendig konstruiert. Die Antriebswelle ist zweiteilig ausgebildet. Zum Festlegen der Welle nach der Entkoppelung von der Walze wird eine gesonderte Vorrichtung benötigt, die aus Federvorrichtungen, Halterungsstangen usw. besteht, die ein rasches und präzises Ein- und Auskoppeln von Antriebswelle und Walzwerkswalze fraglich erscheinen lassen.

Aus der US-PS 2,909,045 ist ein Walzwerksantrieb mit zwischen Kammwalzen und Arbeitswalzen axial verschieblich angeordneten Gelenkspindel bekannt. Das Spindelgelenk ist lösbar mit dem Laufzapfen wenigstens einer Walze, insbesondere der Arbeitswalze verbunden. Das mit der Arbeitswalze verbundene Spindelgelenk weist eine Ein- und Entkopplungsvorrichtung auf, die als Ring-Bajonett-Verschluß ausgebildet ist. Der Bajonett-Verschluß koppelt den Laufzapfen der Arbeitswalze mit der Gelenkhülse des Spindelgelenks. Die Innenverzahnung der Gelenkhülse wirkt mit einer Bogenverzahnung eines auf der Spindelwelle angeordneten Gelenktreffers zusammen. Der Bajonett-Verschluß ist außen an der Gelenkhülse angebracht. Dessen Verschlußrad greift in eine in den Laufzapfen der Walze eingefräste Ausnehmung ein, was zu einer erheblichen Schwächung der Walze an dieser Stelle in folge zusätzlicher Kerbspannungen führt.

Aus der GB-OS 2 187 820 ist eine Klauenkupplung zur Verbindung einer Welle und einer Kardanwelle bekannt. Auf dem Wellenende ist eine Übertragungshülse aufgeschrumpft. In die Stirnseite der Hülse greifen die Zahnklauen des Gelenkkopfes des Kardangelenks ein. In diesem Gelenkkopf ist ein verstellbares Verschlußrad angeordnet, welches die Übertragungshülse mit Spannzähnen hintergreift. Die axiale Verspannung von Übertragungshülse und Verschlußrad wird von einem Schneckengetriebe bewirkt, dessen Achse von außen gedreht werden kann. Diese Bauart einer Klauenkupplung kommt für einen Einsatz im Walzwerksbau deshalb nicht in Frage, weil von dieser Kupplung im Vergleich zu den großen im Walzgerüst zu übertragenden Momenten und Kräften nur geringe Belastungen aufgenommen werden können und die Verstellung des Verschlußrades nicht schnell und nicht präzise genug erfolgen kann.

Ausgehend von der US-PS 2,909,045 ist es die Aufgabe der Erfindung, den Walzwerksantrieb der vorbekannten Gattung in der Weise zu verbessern, daß durch eine einfache, kompakte und sehr genau einstellbare Konstruktion das Auswechseln der Arbeitswalzen bzw. die Trennung von Arbeitswalze und Gelenkspindel erleichtert wird und schneller als bisher durchgeführt werden kann, wobei besonders hohe Anforderungen an die Präzision des Auswechselns gestellt werden, um Walzenlagerung und Spindelgelenk so gering wie möglich durch den Wechselvorgang zu belasten.

Diese Aufgabe wird erfindungsgemäß dadurch gelöst, daß an der Stirnseite des Laufzapfens der Arbeistwalze zentrisch und achsfluchtend ein eine Außenverzahnung aufweisender Verschlußzapfen angeordnet ist, in den ein mit der Gelenkhülse verbundenes und in der Gelenkhülse drehbar gehaltenes mit einer Innenverzahnung versehenes Verschlußrad eingreift, wobei die Außenverzahnung des Verschlußzapfens so hinterschnitten ist, daß die Hinterschneidung eine Ringnut bildet, in der die Innenverzahnung des Verschlußrades in der Weise verdrehbar geführt ist, daß zum Einkuppeln die Innenzähne des Verschlußrades axial fluchtend und formschlüssig hinter die Außenzähne des Verschlußzapfens verdrehbar sind, und daß zum Entkuppeln die Innenzähne des Verschlußrades axial fluchtend zu den jeweiligen Zahnlücken der Außenverzahnung des Verschlußzapfens verdrehbar sind und daß ferner das Verschlußrad eine Außenverzahnung aufweist, in die eine die Drehbewegung des Verschlußrades bewirkende Verstellvorrichtung eingreift, wobei die Verstellvorrichtung mindestens einen außen verzahnten Stößel aufweist, der in einer Führungsbohrung innerhalb der Gelenkhülse etwa tangential zum Verschlußrad geführt ist und von außerhalb der Gelenkhülse verstellbar ist, wobei der Verstellweg des Stößels nach Maßgabe des geforderten Verdrehwinkels α zwischen Verschlußzapfen und Verschlußrad einstellbar ist.

Infolge der erfindungsgemäßen Detaillösung für die Ein- und Entkopplungsvorrichtung innerhalb des Spindelgelenks wird eine sehr kompakte Bauweise geschaffen. Durch die gewonnene Präzision und Genauigkeit der mechanisch lösbaren Verbindung von Walze und Gelenkspindel werden am Walzenlager und an den Übertragungselementen des Spindelgelenks unkontrollierte Belastungen vermieden, so daß Lebensdauer und Verfügbarkeit dieser Konstruktionselemente nicht beeinträchtigt werden. Die vorgeschlagenen Maßnahmen lassen eine genaue axiale Festlegung von Laufzapfen zum Spindelgelenk zu und damit eine exakte Ausrichtung der Walzenachse zur Spindelachse. Dies ist unter anderem Voraussetzung dafür, daß nach einem Auswechseln der Walzen die hohen Drehmomente von der Spindelwelle über Gelenktreffer und Gelenkhülse sicher auf die Walze übertragen werden können. Der im wesentlichen runde Stößel der Verstellvorrichtung weist auf seinem Außenmantel eine Verzahnung in Form einer Zahnstange auf, die so in die Außenverzahnung des Verschlußrades eingreift, daß ein Verschieben des Stößels um eine bestimmte Weglänge eine genau zugeordnete Verdrehung des Verschlußrades bewirkt. Auf diese Weise wird erreicht, daß die Stellung der Verzahnung von Verschlußzapfen bzw. Verschlußrad stets und genau der Öffnungs- bzw. Schließposition des Bajonettverschlusses entspricht.

In Ausgestaltung der Erfindung wird zur genauen Einstellung und Begrenzung des Verstellweges des Zahnstößels zweckmäßigerweise vorgeschlagen, daß der Zahnstößel einen Hohlzylinder mit innenliegendem und von Tellerfedern umgebenen Stößelschaft aufweist, der seinerseits in der Führungsbohrung der Gelenkhülse in einem fest angeordneten Anschlag für den Hohlzylinder gleitend gehalten ist. Erfindungsgemäß ist das Bewegungsspiel zwischen Zahnstößel, vorzugsweise zwischen dessen Hohlzylinder und dem Anschlag nach Maßgabe des geforderten Verdrehwinkels zwischen Verschlußzapfen und Verschlußrad einstellbar.

Schließlich ist in Weiterbildung der Erfindung vorgesehen, daß von der vorgespannten Federvorrichtung das Bewegungsspiel zwischen dem unteren Rand des Hohlzylinders und dem Anschlag vorgegeben wird, und daß an der nach außen gerichteten Fläche des Zylinderkopfes eine Verstelleinheit, vorzugsweise eine hydraulische Kolben-Zylinder-Einheit angreift, von der der Zahnstößel gegen die Federkraft bis zum Anschlag verstellbar ist. Mit Vorteil wird durch diese konstruktive Maßnahme erreicht, daß in der von der Federvorrichtung vorgespannten Position des Zahnstößels das Verschlußrad des Bajonettverschlusses so gedreht ist, daß die Innenverzahnung des Verschlußrades axial fluchtend und formschlüssig hinter die Außenzähne des Verschlußzapfens gedreht sind. Wird dagegen der Zahnstößel von der Verstelleinheit gegen die Vorspannkraft der Tellerfedern bis zum Anschlag in die Führungsbohrung gedrückt, wird von der Verzahnung des Zahnstößels das Verschlußrad um einen definierten Winkel so weit verdreht, daß die Innenzähne des Verschlußrades axial fluchtend zu den jeweiligen Zahnlücken der Außenverzahnung des Verschlußzapfens stehen, so daß ein einwandfreies Entkoppeln von Verschlußrad und Verschlußzapfen gewährleistet ist.

Die Erfindung wird anhand eines Ausführungsbeispiels näher erläutert. Es zeigen:
- Figur 1: den im Spindelgelenk angeordneten Ring-Bajonettverschluß im Schnitt,
- Figur 2: einen Schnitt vom geschlossenen Ring-Bajonettverschluß und der Verstellvorrichtung entlang der Linie II-II in Fig. 1,
- Figur 3: einen Schnitt vom geöffneten Ring-Bajonettverschluß und Verstellvorricht ung entlang der Linie II-II in Fig. 1.

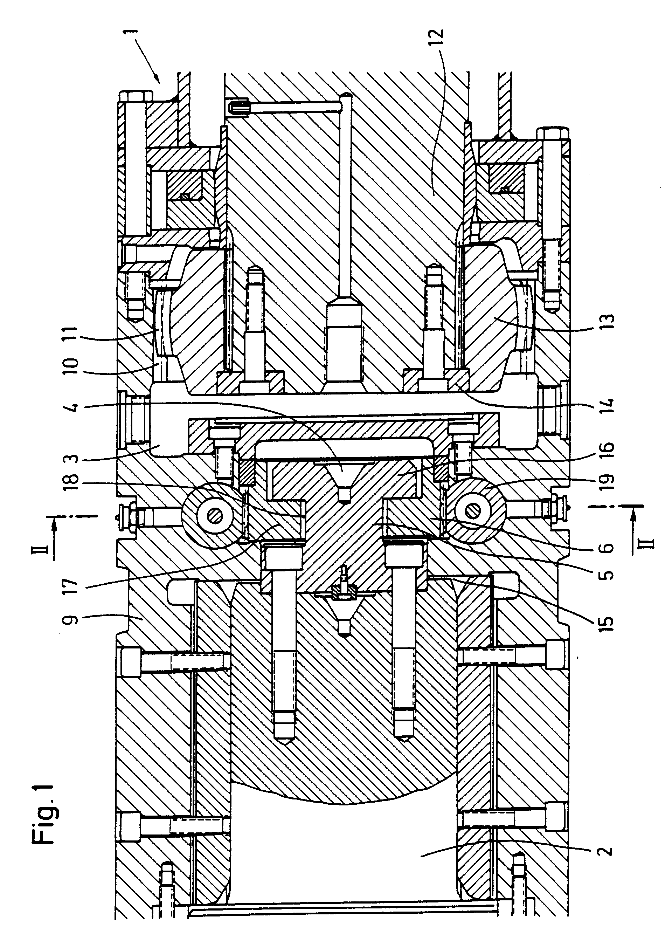

Der Walzwerksantrieb gemäß Fig. 1 besteht aus zwischen nicht näher dargestellten Kammwalzen und Arbeitswalzen angeordneten Gelenkspindeln 1, die axial verschieblich sind und mit den Laufzapfen 2 der Arbeitswalzen lösbar verbunden sind. Die dargestellte Gelenkspindel hat ein Spindelgelenk 3, in welchem zum Ein- und Entkoppeln von Arbeitswalze und Gelenkspindel ein Ring-Bajonettverschluß 4 angeordnet ist.

Der Ring-Bajonettverschluß 4 besteht aus einem Verschlußzapfen 5, einem Verschlußrad 6 und einer Verstellvorrichtung 7, die außerhalb des Spindelgelenks 3 mittels einer Verstelleinheit 8 betätigt wird. Der Ring-Bajonettverschluß 4 koppelt den Laufzapfen 2 der Arbeitswalze mit der Gelenkhülse 9 des Spindelgelenks 3; die Innenverzahnung 10 der Gelenkhülse wirkt mit der Bogenverzahnung 11 des auf der Spindelwelle 12 angeordneten Gelenktreffers 13 zusammen. Der Gelenktreffer 13 ist bspw. mittels einer Keilnutverbindung 14 auf der Spindelwelle 12 drehfest angeordnet.

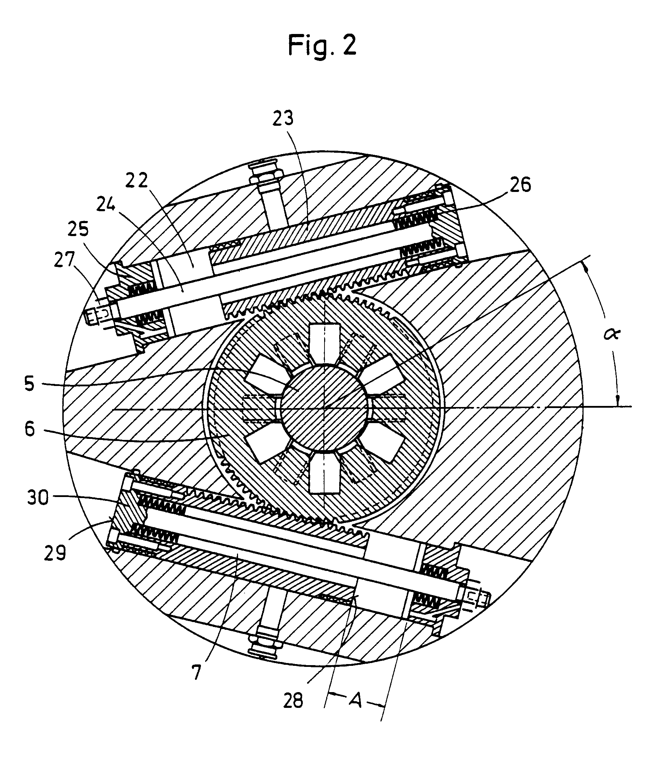

Der Verschlußzapfen 5 des Ring-Bajonettverschlusses 4 ist an der Stirnseite 15 des Laufzapfens 2 der Arbeitswalze zentrisch und achsfluchtend mittels Schraubverbindung befestigt. In den Verschlußzapfen 5 greift das Verschlußrad 6 des Bajonettverschlusses ein, welches in der Gelenkhülse 9 des Spindelgelenks 3 drehbar gehalten ist. Der Verschlußzapfen 5 weist eine Außenverzahnung 16 auf, während das Verschlußrad 6 mit einer Innenverzahnung 17 versehen ist. Die Außenverzahnung 16 des Verschlußzapfens 5 ist so hinterschnitten, daß sich durch die Hinterschneidung eine Ringnut 18 ausbildet, in der das Verschlußrad 6 geführt ist. In der Ringnut 18 ist das Verschlußrad so drehbar, daß bei geschlossenem Bajonettverschluß die Innenzähne 17 des Verschlußrades axial fluchtend und formschlüssig hinter die Außenzähne 16 des Verschlußzapfens 5 gedreht sind (Fig. 2). In dieser Verschlußposition des Bajonettverschlusses 4 ist die Gelenkspindel 1 mit dem Laufzapfen 2 der Arbeitswalze axial fest verbunden. Ferner ist die Gelenkhülse 9 durch eine geeignete und nicht näher dargestellte Verbindung mit dem Laufzapfen 2 radial fest verbunden, so daß die Antriebsmomente von der Spindelwelle 12 über die Gelenktreffer 13 mit Bogenverzahnung 11 und die Gelenkhülse 9 mit Innenverzahnung 10 auf den Laufzapfen 2 und damit auf die Arbeitswalze übertragen werden.

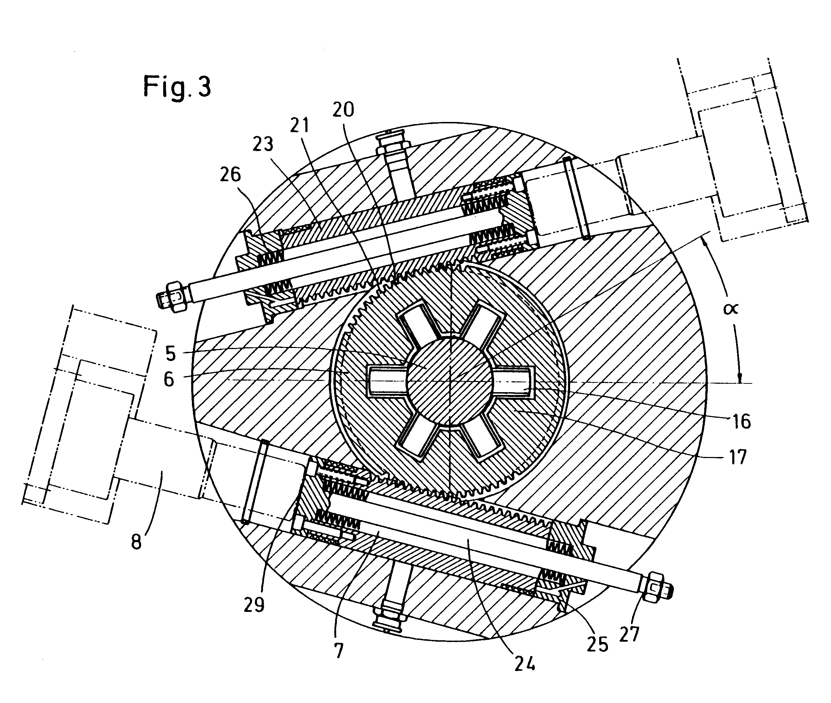

Zum exakten und schnellen Ein- und Auskuppeln des im Spindelgelenk angeordneten Bajonettverschlusses ist gemäß Fig. 2 und Fig. 3 eine besondere Verstellvorrichtung 7 vorgesehen, die ein Betätigen des Bajonettverschlusses von außerhalb der Gelenkhülse ermöglicht. Hierzu weist die Verstellvorrichtung zwei außenverzahnte Stößel 19 auf, deren Verzahnung 20 mit der Außenverzahnung 21 des Verschlußrades 6 im Eingriff stehen. Der Zahnstößel 19 ist in einer Führungsbohrung 22 innerhalb der Gelenkhülse 9 etwa tangential zum Verschlußrad geführt und ist von außerhalb der Gelenkhülse verschiebbar. Der Zahnstößel wird von einem Hohlzylinder 23 und einem Stößelschaft 24 gebildet, der seinerseits in der Führungsbohrung 22 in einem fest angeordneten Anschlag 25 gleitend gehalten ist. Im Hohlzylinder 23 und um den Stößelschaft 24 ist eine von Tellerfedern 26 gebildete Federanordnung vorgesehen. Das Bewegungsspiel A zwischen dem Zahnstößel 19, d.h. zwischen dem unteren Rand 28 des Hohlzylinders 23 und dem Anschlag 25 ist nach Maßgabe des geforderten Verdrehwinkels α zwischen Verschlußzapfen 5 und Verschlußrad 6 des Bajonettverschlusses 4 einstellbar. Die Einstellung erfolgt durch eine am Ende des Stößelschaftes vorgesehene Rändelschraube 27.

Mit Hilfe der Tellerfedern 26 wird der Hohlzylinder 23 gegenüber dem Anschlag 25 auf Abstand gehalten, so daß von der vorgespannten Federanordnung das Bewegungsspiel A zwischen dem unteren Rand des Hohlzylinders und dem Anschlag vorgegeben wird. Die nach außen gerichtete Fläche 29 des Zylinderkopfes 30 ist mit der Verstelleinheit 8 verbindbar, die vorteilhafterweise eine hydraulische Kolben-Zylinder-Einheit ist. Von der Verstelleinheit wird der Zahnstößel 19 gegen die Federkraft der Tellerfedern 26 bis zum Anschlag 25 verschoben.

Gemäß Fig. 3 ist der Zahnstößel 19 von der Verstelleinheit 8 gegen den Anschlag 25 verschoben worden. Es ist erkennbar, daß in dieser Position von dem Zahnstößel das Verschlußrad 6 des Bajonettverschlusses so weit gedreht ist, daß die Innenzähne des Verschlußrades 6 axial fluchtend zu den jeweiligen Zahnlücken der Außenverzahnung des Verschlußzapfens 5 angeordnet sind. In dieser Verstellposition ist der Bajonettverschluß 4 geöffnet, d.h. das Verschlußrad 6 kann von dem Verschlußzapfen 5 in axialer Richtung abgezogen werden. Während des Entkopplungsvorganges bleibt der Zahnstößel 19 gegen den Anschlag 25 verschoben.

Zur erneuten axialen Arretierung von Laufzapfen 2 und Gelenkhülse 9 des Spindelgelenks 3 wird der Zahnstößel 19 von der hydraulischen Verstelleinheit 8 gegen den Anschlag 25 verschoben. Die hierdurch auf Zahnlücke stehende Innenverzahnung des Verschlußrades 6 und Außenverzahnung des Verschlußzapfen 5 ermöglicht es, das Verschlußrad in axialer Richtung über den Verschlußzapfen bis in den Bereich der Ringnut 18 zu verschieben. Sobald das Verschlußrad in der Ringnut 18 geführt ist, wird die hydraulische Verstelleinheit 8 zurückgefahren und der Zahnstößel 19 wird von der Federkraft der Tellerfedern 26 um das fest eingestellte Bewegungsspiel A in seine Ausgangslage zurückgedrückt. Hierdurch wird das Verschlußrad 6 um den vorgegebenen Winkel so gedreht, daß die Innenverzahnung des Verschlußrades axial fluchtend und formschlüssig hinter die Außenzähne des Verschlußzapfens gedreht wird.

Auf diese Weise wird eine schnelle und exakte Verbindung bzw. Trennung von Arbeitswalze und antreibender Gelenkspindel 1 gewährleistet, sobald die Arbeitswalzen aus Verschleißgründen ersetzt werden müssen oder aber die Arbeitswalzen dem jeweiligen Produktionsprogramm angepaßt werden müssen.

### Bezugszeichenübersicht

- 1: Gelenkspindel
- 2: Laufzapfen
- 3: Spindelgelenk
- 4: Ring-Bajonettverschluß
- 5: Verschlußzapfen
- 6: Verschlußrad
- 7: Verstellvorrichtung
- 8: Verstelleinheit
- 9: Gelenkhülse
- 10: Innenverzahnung der Gelenkhülse
- 11: Bogenverzahnung des Gelenktreffers
- 12: Spindelwelle
- 13: Gelenktreffer
- 14: Keilnutverbindung
- 15: Stirnseite des Laufzapfens
- 16: Außenverzahnung des Verschlußzapfens
- 17: Innenverzahnung des Verschlußrades
- 18: Ringnut
- 19: Stößel
- 20: Verzahnung des Stößels
- 21: Außenverzahnung des Verschlußrades
- 22: Führungsbohrung
- 23: Hohlzylinder
- 24: Stößelschaft
- 25: Anschlag
- 26: Tellerfedern
- 27: Rändelschraube
- 28: Hohlzylinderrand
- 29: Zylinderkopffläche
- 30: Zylinderkopf

## Patentansprüche

1. Walzwerksantrieb mit zwischen Kammwalzen und Arbeitswalzen axial verschieblich angeordneten Gelenkspindeln (1), deren Spindelgelenk (3) lösbar mit dem Laufzapfen (2) wenigstens einer Walzen, insbesondere der Arbeitswalze verbunden ist, wobei zumindest das mit der Arbeitswalze verbundene Spindelgelenk (3) eine Ein- und Entkopplungsvorrichtung aufweist, die als Ring-Bajonett-Verschluß (4) ausgebildet ist, wobei der Ring-Bajonett-Verschluß (4) den Laufzapfen (2) der Arbeitswalze mit der Gelenkhülse (9) des Spindelgelenks (3) koppelt und die Innenverzahnung (10) der Gelenkhülse (9) mit einer Bogenverzahnung (11) eines auf der Spindelwelle (12) angeordneten Gelenktreffers (13) zusammenwirkt,
**dadurch gekennzeichnet,**
daß an der Stirnseite (15) des Laufzapfens (2) der Arbeitswalze zentrisch und achsfluchtend ein eine Außenverzahnung (16) aufweisender Verschlußzapfen (5) angeordnet ist, in den ein mit der Gelenkhülse (9) verbundenes und in der Gelenkhülse drehbar gehaltenes mit einer Innenverzahnung (17) versehenes Verschlußrad (6) eingreift, wobei die Außenverzahnung (16) des Verschlußzapfens (5) so hinterschnitten ist, daß die Hinterschneidung eine Ringnut (18) bildet, in der die Innenverzahnung (17) des Verschlußrades (6) in der Weise verdrehbar geführt ist, daß zum Einkuppeln die Innenzähne (17) des Verschlußrades (6) axial fluchtend und formschlüssig hinter die Außenzähne (16) des Verschlußzapfens (5) verdrehbar sind und daß zum Entkuppeln die Innenzähne (17) des Verschlußrades (6) axial fluchtend zu den jeweiligen Zahnlücken der Außenverzahnung (16) des Verschlußzapfens (5) verdrehbar sind und daß ferner das Verschlußrad (6) eine Außenverzahnung (21) aufweist, in die eine die Drehbewegung des Verschlußrades (6) bewirkende Verstellvorrichtung (7) eingreift, wobei die Verstellvorrichtung (7) mindestens einen außen verzahnten Stößel (19) aufweist, der in einer Führungsbohrung (22) innerhalb der Gelenkhülse etwa tangential zum Verschlußrad (6) geführt ist und von außerhalb der Gelenkhülse verstellbar ist, wobei der Verstellweg des Stößels (19) nach Maßgabe des geforderten Verdrehwinkels α zwischen Verschlußzapfen (5) und Verschlußrad (6) einstellbar ist.

2. Walzwerksantrieb nach Anspruch 1,
**dadurch gekennzeichnet,**
daß der Stößel (19) einen Hohlzylinder (23) mit innen liegendem und von einer Federanordnung, vorzugsweise von Tellerfedern, umgebenen Stößelschaft (24) aufweist, der seinerseits in der Führungsbohrung (22) der Gelenkhülse (9) in einem fest angeordneten Anschlag (25) für den Hohlzylinder gleitend gehalten ist.

3. Walzwerksantrieb nach Anspruch 1 oder 2,
**dadurch gekennzeichnet,**
daß das Bewegungsspiel (A) zwischen Stößel (19), vorzugsweise zwischen dessen Hohlzylinder (23) und dem Anschlag (25) nach Maßgabe des geforderten Verdrehwinkels α zwischen Verschlußzapfen (5) und Verschlußrad (6) einstellbar ist.

4. Walzwerksantrieb nach Anspruch 1, 2 oder 3,
**dadurch gekennzeichnet,**
daß von der vorgespannten Federvorrichtung das Bewegungsspiel (A) zwischen dem unteren Rand (28) des Hohlzylinders (23) und dem Anschlag vorgegeben wird und daß an der nach außen gerichteten Fläche (29) des Zylinderkopfes eine Verstelleinheit (8), vorzugsweise eine hydraulische Kolben-Zylinder-Einheit angreift, von welcher der Stößel (19) gegen die Federkraft bis zum Anschlag (25) verstellbar ist.

## Claims

1. Rolling mill drive with articulated spindles (1), which are arranged between gear rolls and work rolls and the spindle joint (3) of which is detachably connected with the neck pin (2) of at least one roll, in particular the work roll, wherein at least the spindle joint (3) connected with the work roll comprises a coupling and decoupling device, which is constructed as an annular bayonet lock (4), wherein the annular bayonet lock (4) couples the neck pin (2) of the work roll with the joint sleeve (9) of the spindle joint (3) and the internal toothing (10) of the joint sleeve (9) cooperates with a curved toothing (11) of a joint wobbler (13) arranged on the spindle shaft (12), characterised thereby that centrally and coaxially arranged at the end face (15) of the neck pin (2) of the work roll is a lock pin (5), which has an external toothing (16) and in which engages a lock wheel (6) which is connected with the joint sleeve (9), is rotatably retained in the joint sleeve and is provided with an internal toothing (17), wherein the external toothing (16) of the lock pin (5) is so undercut that the undercutting forms an annular groove (18) in which the internal toothing (17) of the lock wheel (6) is rotatably guided in such a manner that for the coupling-in the internal teeth (17) of the lock wheel (6) are rotatable into axial alignment with and shape-lockingly behind the external teeth (16) of the lock pin (5) and that for the decoupling the internal teeth (17) of the lock wheel (6) are rotatable into axial alignment with the respective tooth gaps of the external toothing (16) of the lock pin (15) and that further the lock wheel (6) has an external toothing (21), in which engages an adjusting device (7) effecting the rotational movement of the lock wheel (6), wherein the adjusting device (7) comprises at least one outer toothed drive plunger (19), which is guided in a guide bore (22), within the joint sleeve approximately tangentially to the lock wheel (6) and which is adjustable from outside the joint sleeve, wherein the adjusting path of the drive plunger (19) is settable in proportion to the required turning angle α between the lock pin (5) and the lock wheel (6).

2. Rolling mill drive according to claim 1, characterised thereby that the drive plunger (19) comprises a hollow cylinder (23) with a plunger shaft (24), which is surrounded by a spring arrangement, preferably by plate springs, and which in turn is slidingly retained in the guide bore (22) of the joint sleeve (9) in a fixedly arranged abutment (25) for the hollow cylinder.

3. Rolling mill drive according to claim 1 or 2, characterised thereby that the movement play (A) between drive plunger (19), preferably between the hollow cylinder (23) thereof, and the abutment (25) is settable in proportion to the required turning angle α between the lock pin (5) and the lock wheel (6).

4. Rolling mill drive according to claim 1, 2 or 3, characterised thereby that the movement play (A) between the lower edge (28) of the hollow cylinder (23) and the abutment is preset by the prestressed spring arrangement and that an adjusting unit (8), preferably a hydraulic piston-cylinder unit, by which the drive plunger (19) is adjustable against the spring force up to the abutment (25), engages at the outwardly directed surface (29) of the cylinder head.

## Revendications

1. Entraînement pour laminoir à allonges d' entraînement (1) déconnectables dans le sens axial entre roues dentées et cylindres de travail, dans lesquelles l'articulation d'allonge (3) est reliée d'une manière amovible au tourillon (2) de l'un au moins des cylindres, de préférence du cylindre de travail, celle au moins des articulations d'allonge (3) qui est reliée au cylindre de travail comprenant un dispositif d'accouplement et de désaccouplement constitué par une fermeture à baïonnette annulaire (4), cette fermeture à baïonnette annulaire (4) accouplant le tourillon (2) du cylindre de travail au manchon d'articulation (9) de l'articulation d'allonge (3) et l'endentement intérieur (10) du manchon d'articulation (9) coopérant avec un accouplement (11) en forme d'arc d'un élément d'articulation (13) monté sur l'arbre d'allonge (12), caractérisé en ce qu'au niveau du côté frontal (15) du tourillon (2) du cylindre de travail le dispositif comprend un tourillon (5) du cylindre de travail qui est centré et dans l'alignement de l'axe et comporte un endentement extérieur (16) et dans lequel est en prise une roue de fermeture (6) qui est reliée au manchon d'articulation (9), est maintenue de manière à pouvoir tourner dans le manchon d'articulation et comporte un endentement intérieur (17), l'endentement extérieur (16) du tourillon de fermeture (5) étant découpé de telle manière que ce decoupage donne une rainure annulaire (18) dans laquelle l'endentement intérieur (17) de la roue de fermeture (6) est guidé de manière à pouvoir tourner et en ce que, pour l'accouplement, les dents intérieures (17) de la roue de de fermeture (6) peuvent tourner pour se placer axialement dans l'alignement et s'engager avec adaptation des formes en arrière des dents extérieures (16) du tourillon de fermeture (5) et en ce que, pour le désaccouplement, les dents intérieures (17) de la roue de fermeture (6) peuvent tourner pour se placer axialement dans l'alignement des creux de dents correspondants de l'endentement extérieur (16) du tourillon de fermeture (5) et en ce que, de plus la roue de fermeture (6) comporte un endentement extérieur dans lequel est en prise un dispositif de déplacement (7) qui produit la rotation de la roue de fermeture (6), ce dispositif de déplacement (7) comprenant au moins un poussoir (19) denté extérieurement qui est guidé dans un alésage de guidage (22) à l'intérieur du manchon d'articulation, dans une direction sensiblement tangentielle à la roue de fermeture (5) et pouvant être déplacé depuis l'extérieur du manchon d'articulation, le trajet de déplacement du poussoir (19) étant réglable d'après la valeur que doit avoir l'angle de déviation α entre le tourillon de fermeture (5) et la roue de fermeture (6).

2. Entraînement pour laminoir selon la revendication 1, caractérisé en ce que le poussoir (19) comporte un cylindre creux (23) contenant une tige de poussoir (24) entourée par un dispositif à ressort, de préférence à ressorts Belleville, qui, de son côté, est maintenu de manière à pouvoir glisser dans l'alésage de guidage (22) du manchon d'allonge (9) contre une butée fixe (25) associée au cylindre creux.

3. Entraînement pour laminoir selon l'une des revendications 1 ou 2, caractérisé en ce que le jeu de déplacement (A) entre le poussoir (19), de préférence entre son cylindre creux (23), et la butée (25) peut être réglé suivant la valeur que doit avoir l'angle de déviation α entre le tourillon de fermeture (5) et la roue de fermeture (6).

4. Entrzînement pour laminoir selon l'une des revendications 1, 2 ou 3 caractérisé en ce que c'est le dispositif à ressort précontraint qui détermine le jeu de déplacement (A) entre le bord inférieur (28) du cylindre creux (23) et la butée et en ce qu'au niveau de la surface (29) de la tête de cylindre qui est tournée vers l'extérieur le dispositif comporte une unité de déplacement (8), de préférnece une unité à cylindre et piston, qui peut, en s'opposant à la force du ressort, déplacer le poussoir (19) jusqu'à la butée (25).
